# EUROPEAN PATENT SPECIFICATION

(11) **EP 0 470 649 B1**
(45) Date of publication and mention of the grant of the patent: **02.06.1999**
(21) Application number: 91117022.3
(22) Date of filing: 23.07.1987
(51) Int. Cl.: G01N 27/42, G01N 27/416

(54) **Method for electrochemical measurements**
Verfahren für elektrochemische Messungen
Méthode pour des mesures électro-chimiques

(30) Priority: 23.07.1986 GB 8618022
(43) Date of publication of application: 12.02.1992
(62) Divisional of application: 87306513.0
(73) Proprietor: UNILEVER PLC, London EC4P 4BQ (GB); UNILEVER N.V., 3013 AL Rotterdam (NL)
(72) Inventor: Birch, Brian Jeffrey, Northamptonshire NN9 6AW, England (GB); Burns, Ian William, Cambridgeshire PE18 0NP, England (GB)
(74) Representative: Butler, David John

(56) References cited:
- EP-A- 0 170 375
- US-A- 3 904 487
- US-A- 4 059 406
- US-A- 4 374 041

## Description

### Field of the Invention

This invention relates to a method for making electrochemical measurements, in particular but not exclusively for the purpose of carrying out microchemical testing on small liquid samples of biological, e.g. clinical origin.

### Background to the Invention

F. Schlapfer et al (Clin.Chim.Acta, 1974, pp 283-289) described electrochemical measurement of glucose concentration using glucose oxidase and soluble electron transfer substances such as ferricyanide, p-benzoquinone, 2,6-dichlorophenolindophenol, pyocyanine, thionine or methylene blue, interacting with amperometric noble-metal electrodes.

These arrangements have not given rise to glucose-measurement products which are simple and convenient to use in environments far removed from the skilled inhabitants of analytical laboratories.

Since 1974 a variety of further electrode arrangements have been proposed for chemical/immunochemical analysis, among them electrodes carrying immobilised redox mediators as well as enzymes, for example EP 0078636, 0125136 and 0125139 (Genetics International), and 0142301 (Serono). EP 0177743 (Shimadzu) describes enzyme electrodes of somewhat complex construction, which are capable of use to measure a number of enzyme substrates by amperometry, using electron transfer mediators.

EP 0125137 discloses an electrode sensing system, for monitoring components in a liquid mixture, using a probe-type sensor which typically comprises an electrode surface carrying a generally insoluble electron transfer mediator, in turn coated with an enzyme, the electron transfer mediator acting to transfer charge between the enzyme and the electrode. The system is typically used to determine glucose concentrations, using amperometric techniques.

The paper in "Analytical Chemistry", Volume 56, No. 2, February 1984, pages 148 to 152 discloses an enzyme electrode using a thin layer platinum electrode in conjunction with the enzyme glucose oxidase or flavine adenine dinucleotide, and refers to the possibility of reducing the depth of the reaction chamber to optimize performance.

US 4059406 discloses an electrochemical detector having a thin layer flow cell with a sensor electrode, a counter electrode and a reference electrode. A solution to be monitored is supplied to the cell by a wall-jet arrangement and a periodically changing voltage is applied to the sensor electrode, so that a reaction product is deposited on the electrode, and is then subsequently removed. Typical applications for the detector include continuous monitoring of trace electroactive materials in water, continuous flow anodic stripping voltametry and organic electro analytical chemistry.

This invention aims to provide a method to enable quick, convenient and accurate measurement of various constituents of liquid samples, especially of biological origin, e.g. clinical samples of blood, serum, or urine.

Within this context we describe a measurement apparatus of simple construction which can be treated as disposable.

It is a further aim of the present invention to provide a method using electrochemical cells for convenient liquid sample analysis but without requiring complex electrode structures involving immobilised components.

### Summary of the Invention

According to one aspect of the invention there is provided a method for measurement or detection of a component of an aqueous liquid sample, said method comprising:
placing a sample, possibly containing the component of interest, into a reaction cell, said reaction cell comprising a capillary fill device and at least two electrodes having an impedance which renders them suitable for coulometric, amperometric or voltammetric processes, so that the sample forms a layer of liquid having a thickness less than about 0.2 millimetres in a reaction zone overlying one of said electrodes,
reacting said component, if present, directly or indirectly with a redox reagent represented by one of said electrodes to cause deposition of a derivative of said component on said one electrode, characterised by
monitoring current on subsequent stripping of said derivative to provide an indication of the presence or quantity of said component of interest by coulometry, voltammetry or amperometry.

The result relating to the quantity of the substance to be measured can be of use, among other things, as an index of the concentration of the substance in a liquid sample.

In this specification and claims 'redox reagent' and similar terms and 'electron transfer reagent' and corresponding terms are mutually inclusive.

The invention may be performed in apparatus for carrying out electrochemical detection or measurement of a component of an aqueous liquid sample, said apparatus comprising:
a reaction cell comprising at least two electrodes, the electrodes having an impedance which renders them suitable for coulometric, amperometric or voltammetric processes and being located in or adjacent to a reaction zone, said reaction zone being capable of receiving an aqueous liquid sample possibly containing said component of interest, and said electrodes and said reaction zone being arranged so that liquid in said zone contacts said electrodes and forms a layer having a thickness less than about 0.2 millimetres overlying one of said electrodes,
and said cell comprising a redox reagent represented by one of said electrodes so that a derivative of said component is deposited on said one electrode on application of a suitable electrical potential, and means for monitoring current on subsequent stripping of said derivative to provide an indication of the presence or quantity of said component of interest by coulometry, voltammetry or amperometry.

It is especially preferred to provide in the use of this method a cell which confines the liquid reagents to react with the electrode to a sufficiently thin layer overlying the electrode to permit measurement to take place in a short time. A suitable thickness for the liquid layer is for example of the order of about 0.02 to 0.2 mm, for example about 0.1 mm. Capillary-fill cells with a configuration as described in EP 0170375 (Unilever) are among the cells suitable in this respect. In certain useful arrangements within the scope of the invention, the cell may confine a defined reactive volume of sample or reaction liquid in a space of defined width between a cell wall and an electrode of defined area. Liquid outside the volume maybe able to diffuse inwards but for example only at an inappreciable rate compared to the time required for reaction of the liquid in the defined volume. In other useful embodiments, the cell may define a volume of liquid to provide material to react at the electrode.

In one test which can be carried out using the device and method described in more detail herein, the component to be measured comprises an electrochemically reducible metal ion, and is measured by anodic stripping.

The form of the reaction cell in which the reaction is allowed to take place can contribute significantly to the convenience of the test procedure. It is preferred to use an adapted form of the capillary fill cells provided with electrodes as described in European Specification No. 0170375 (Unilever) (see prior art portion of claim 1), containing electrodes of suitable impedance carried as thin films on one or more walls thereof.

Such a cell as adapted for the purposes of the present invention can suitably for example comprise three electrodes, viz (a) a working electrode, for example of gold or other noble metal, carbon or graphite in any convenient form, e.g. wax-impregnated graphite; (b) a counterelectrode, chosen from a similar range of materials as given for electrode (a), and possibly of the same material as electrode (a) itself; and (c) a reference electrode, for example a silver/chloride electrode, or pH electrode.

Choice of electrode materials can for many purposes preferably be made among gold, silver and carbon film electrodes. Example of preferred uses are: gold electrodes are very suitable for voltammetry of trace quantities of mercury, and gold electrodes in combination with a thin mercury layer, e.g. produced by preliminary electrochemical deposition from a quantity of mercuric salt, are very suitable for analysis of heavy metals or organic materials such as drugs, e.g. morphine. Carbon electrodes are very suitable e.g. for electroanalysis of oxidisable organic groups such as amines, or reducible organic groups such a nitrobenzenes. The aim is to provide electrodes with low enough impedance for amperometric or voltammetric use, in a manner well known in itself.

A preferred configuration involves the use of a cell comprising a pair of gold electrodes. In this case one gold electrode can serve as a counter-electrode as well as a reference electrode.

In one convenient form, both or all electrodes are contained as films in a capillary fill cell formed between two parallel flat plates spaced apart by about 0.1 mm cell thickness, with about 0.1 mm thick tracks of sealing material forming the remaining sides of the cell apart from an aperture for entry of liquids.

It can be convenient to form such a cell using opposed plates of for example ceramic, plastics or glass. When such a cell is fabricated, as is preferred, by the use of ceramics, a suitable substrate can be for example a 96% alumina substrate (Kyocera A4476-Trade Mark), and a preferred material for the electrodes to be formed thereon is gold, applied as gold printing paste (Engelhard T4474-Trade Mark), to be applied in a high temperature oxidative furnace in accordance with the ordinary methods of use of that material. This results in the context of this invention in a gold layer with an overlying thin oxide layer capable of constituting a highly reproducible electrode. Whenever desired, part of the metal layer can be blanked off by overlying dielectric layers e.g. formed of dielectric printing ink (DuPont 5704 - Trade Mark) applied to the substrate according to the ordinary manner of use of that material.

When reactions of the kinds described above are allowed to occur in a cell as described above, it is found that a working electrode can easily deplete substantially all of the electroactive material in that part of the liquid that overlies the working electrode, before any substantial lateral diffusion has taken place.

Accordingly, it is preferred to use cells of such dimensions that this situation prevails: i.e. that the time required for lateral diffusion of an appreciable amount of reactive material from the region outside that which overlies the working electrode, to the region overlying the working electrode, is much longer than the time required for diffusion of cell contents across the thickness of the cell and for depletion by an electrode of the material capable of reacting with it from the region of the cell overlying said electrode.

An advantage arising from use of the invention in this manner is that calibration of the measurements can be particularly simple and uniform as between samples of the device as described herein.

Further details of the invention are given below in connection with the following illustrative example.

### Example

The process of the invention can be used for trace metal analysis by anodic stripping voltammetry. In this arrangement, it can be convenient to use a noble metal or (preferably) carbon working electrode, pH buffer, and excess mercuric nitrate. In a calibration test, for example, the test liquid in the cell can contain 1 microgram/ml each of cadmium, lead and copper ions, and 20 microgram/ml mercuric ions, in acetate buffer 0.1M pH 4.8. The measurement method is as follows. After introducing the reagents into a capillary fill cell provided with electrodes as described above, (a) first a high negative potential (-1.0 volt) can be applied to the working electrode for a precisely standardised time in this instance chosen as 30 seconds, to deposite Hg metal thereon, to form a thin mercury film including as amalgam whatever traces of metals may deposit together with the mercury. When a capillary fill cell is used, complete deposition may be achieved and standardisation of time may be rendered unnecessary.

Thereafter, (b) a positive going potential ramp is applied to the working electrode (e.g. from -1.0 volt to +0.1 volt using a differential pulse technique at an overall scan rate of 10 mV per second with +50 mV pulse height using a PAR 264 polarograph (Trade Mark) from Princeton Applied Research). Measurement of the resulting current is made to determine the occurrence of any peaks. Any peaks that occur indicate the presence of a metal identifiable in principle by the potential at which the peak is observed, in a quantity corresponding to the area under or height of the current peak.

The reagents can be dried down and deoxygenating reagents such as ascorbic acid and/or sodium sulphite can be included. Alternatively an a.c. polarographic technique can be used with no need for the salts to be added.

It is of course possible to carry out measurements of other substances by stripping voltammetry using appropriate electrodes, as hitherto described.

Thus, possible variants of the Example are:-
(a) Nickel can be estimated using a cell containing dimethylglyoxime as follows: In the cell there is provided as a reagent layer (together with mercuric acetate or nitrate) a layer of dimethylglyoxime salt, pH 7. This reacts with nickel (2+) introduced in a sample to form a complex. At about -0.4 volt mercury deposits on the (e.g. gold or carbon) electrodes with an oxidised form of the nickel complex. The complex can then be stripped and analysed using a negatively going potential gradient.
In this variant, preferred electrode systems are either a 3-electrode system comprising two gold electrodes and a silver reference electrode, or a 2-electrode system comprising a gold electrode and a silver (chloride) sacrificial electrode.
(b) Organic materials, e.g. drugs such as morphine, can be estimated by absorptive stripping voltammetry using cell arrangements as described herein: holding a gold or carbon electrode at a negative potential for about a minute, and by holding the electrode at about 0 volt for about a minute the morphine may be absorbed: on reducing the electrode potential past about -0.2 volts the morphine derivative is stripped off from the working electrode and generates a current peak from the amount of which the result of the test may be obtained.

In further embodiments, it can be sufficient to use a working electrode and a further electrode combining the functions of reference and counter electrode, provided that the further electrode is a metal/metal-halide reference electrode and the corresponding halide is present in the sample, preferably at standardised high concentration. Alternatively, any other further electrode of low electrochemical impedance and adequately-defined potential may be used. Suitable electrodes for use are for example as described in EP 0186286 (Unilever).

The invention described herein is susceptible of many modifications and variations as will be apparent to the skilled reader, and the disclosures herein extend to the scope of the invention as claimed.

## Claims

1. A method for measurement or detection of a component of an aqueous liquid sample, said method comprising:
placing a sample, possibly containing the component of interest, into a reaction cell, said reaction cell comprising a capillary fill device and at least two electrodes having an impedance which renders them suitable for coulometric, amperometric or voltammetric processes, so that the sample forms a layer of liquid having a thickness less than about 0.2 millimetres in a reaction zone overlying one of said electrodes,
reacting said component, if present, directly or indirectly with a redox reagent represented by one of said electrodes to cause deposition of a derivative of said component on said one electrode, characterised by
monitoring current on subsequent stripping of said derivative to provide an indication of the presence or quantity of said component of interest by coulometry, voltammetry or amperometry.

2. A method according to claim 1, wherein said component to be measured comprises an electrochemically reducible ion, an electrochemically oxidizable inorganic ion or an electrochemically oxidizable or reducible organic compound

3. A method according to claim 1 or 2, wherein a trace metal is analysed by anodic stripping voltammetry.

4. A method according to claim 1 or 2, wherein organic materials are estimated by absorptive stripping voltammetry.

## Patentansprüche

1. Verfahren zur Messung oder zum Nachweis eines Bestandteiles einer wäßrigen flüssigen Probe, wobei das Verfahren umfaßt:
Einführen einer Probe, die den interessierenden Bestandteil möglicherweise enthält, in eine Reaktionszelle, wobei die Reaktionszelle eine kapillarförmige Füllvorrichtung umfaßt und mindestens zwei Elektroden, die eine Impendanz aufweisen, die sie für coulometrische, amperometrische oder voltametrische Verfahren geeignet macht, so daß die Probe eine Flüssigkeitsschicht mit einer Dicke von weniger als etwa 0,2 mm in einer Reaktionszone bildet, die eine der Elektroden überschichtet,
Umsetzen des Bestandteils, falls vorhanden, direkt oder indirekt mit einem Redoxreagenz, das von einer der Elektroden verkörpert wird, um die Ablagerung eines Derivates des Bestandteils auf der Elektrode zu bewirken, gekennzeichnet durch
Überwachen des Stromflusses beim anschließenden Ablösen des Derivats, um eine Anzeige der Gegenwart oder der Menge des interessierenden Bestandteils durch Coulometrie, Voltametrie oder Amperometrie bereitzustellen.

2. Verfahren gemäß Anspruch 1, worin der zu messende Bestandteil ein elektrochemisch reduzierbares Ion, ein elektrochemisch oxidierbares anorganisches Ion oder eine elektrochemisch oxidierbare oder reduzierbare organische Verbindung umfaßt.

3. Verfahren gemäß Anspruch 1 oder 2, worin ein Spurenmetall durch anodische Ablösevoltametrie analysiert wird.

4. Verfahren gemäß Anspruch 1 oder 2, worin organische Materialien durch absorptive Ablösevoltametrie abgeschätzt werden.

## Revendications

1. Procédé de mesure ou de détection d'un composant d'un échantillon liquide aqueux, ledit procédé comportant les étapes consistant à :
◆ placer un échantillon, contenant peut-être le composant d'intérêt, dans une cellule de réaction, ladite cellule de réaction comportant un dispositif de remplissage capillaire et au moins deux électrodes ayant une impédance qui les rend adaptées à des processus coulométrique, ampérométrique ou voltamétrique, de sorte que l'échantillon forme une couche de liquide ayant une épaisseur inférieure à environ 0,2 millimètre dans une zone de réaction qui est sus-jacente à une première desdites électrodes,
◆ faire réagir ledit composant, s'il est présent, directement ou indirectement à l'aide d'un réactif d'oxydoréduction représenté par une première desdites électrodes pour entraîner un dépôt d'un dérivé dudit composant sur ladite première électrode, caractérisé par l'étape consistant à :
• contrôler le courant lors d'une séparation ultérieure dudit dérivé pour fournir une indication de la présence ou de la quantité dudit composant d'intérêt par coulométrie, voltamétrie ou ampérométrie.

2. Procédé selon la revendication 1, dans lequel ledit composant à mesurer comporte un ion électrochimiquement réductible, un ion inorganique électrochimiquement oxydable ou un composé organique électrochimiquement réductible ou oxydable.

3. Procédé selon la revendication 1 ou 2, dans lequel un métal à l'état de trace est analysé par voltamétrie par séparation anodique.

4. Procédé selon la revendication 1 ou 2, dans lequel des matériaux organiques sont estimés par voltamétrie d'absorption par séparation.
